# EUROPÄISCHE PATENTSCHRIFT

(11) **EP 0 847 279 B1**
(45) Veröffentlichungstag und Bekanntmachung des Hinweises auf die Patenterteilung: **02.05.2002**
(21) Anmeldenummer: 96930055.7
(22) Anmeldetag: 19.08.1996
(51) Int. Cl.: A61K 45/06

(54) **JUCKREIZLINDERNDE, KOSMETISCHE UND/ODER PHARMAZEUTISCHE ZUSAMMENSETZUNGEN BESTEHEND AUS EINEM ODER MEHREREN LEICHTEN LOKAL ANÄSTHETIKA UND EINER ODER MEHREREN ADSTRINGENTIEN**
ANTIPRURIGINOUS COSMETIC AND/OR PHARMACEUTICAL COMPOSITIONS CONSISTING OF ONE OR SEVERAL LIGHT LOCAL ANAESTHETICS AND ONE OR SEVERAL ASTRINGENT SUBSTANCES
COMPOSITIONS COSMETIQUES ET/OU PHARMACEUTIQUES ANTIPRURIGINEUSES CONTENANT UN OU PLUSIEURS ANESTHESIQUES LOCAUX LEGERS ET UN OU PLUSIEURS ASTRINGENTS

(30) Priorität: 30.08.1995 DE 19531893
(43) Veröffentlichungstag der Anmeldung: 17.06.1998
(73) Patentinhaber: BAYER AG, 51368 Leverkusen (DE)
(72) Erfinder: RÖDER, Klaus, D-51377 Leverkusen (DE); KOCH, Sabine, D-51467 Bergisch Gladbach (DE)
(86) Internationale Anmeldenummer: EP9603641
(87) Internationale Veröffentlichungsnummer: WO9707821

(56) Entgegenhaltungen:
- AKTUEL. DERMATOL., 1990, 16/8 (221-225), GERMANY, FEDERAL REPUBLIC OF, XP000614542 BERGNER T. ET AL: "Prurigo simplex subacuta"
- PROF CARE MOTHER CHILD, MAY 1994, 4 (4) P109-10, ENGLAND, XP000614706 SINCLAIR A: "Remedies for common family ailments: 4. Insect bites and stings."
- HELWIG H.; HANS-HARTWIG O. : "HELWIG ARZNEIMITTEL" 1988 , STUTTGART, WVG.; DE XP002023751 THESIT siehe Seite 37-13, Spalte 1, Absatz 4

## Beschreibung

Die vorliegende Erfindung betrifft juckreizlindernde kosmetische und/oder pharmazeutische Zusammensetzungen zur Anwendung auf der menschlichen oder tierischen Haut.

Insektenstiche und der damit verbundene Juckreiz sind nicht nur unangenehm, sondern können durch Kratzen an den juckenden Hautpartien zu Entzündungen führen. Die Narben heilen oft schwer ab und sind lange bzw. dauerhaft sichtbar. Die Linderung des Juckreizes ist damit besonders wichtig, um Komplikationen zu vermeiden.

Aus der Literatur ist bekannt, daß ethoxylierte Verbindungen wie z.B. Laureth-9 (Fiedler, Lexikon der Hilfsstoffe für Pharmazie und Kosmetik und angrenzende Gebiete, Verlag: Editio Cantor Aulendorf, 3. Auflage, Band 1, S. 540) oder Polyoxyethylen-(20)-Sorbitanmonolaurat (Herstellerangabe) oder Stoffe aus anderen Substanzklassen z.B. Zitronensäureester (Fiedler, Lexikon der Hilfsstoffe für Pharmazie und Kosmetik und angrenzende Gebiete, Verlag: Editio Cantor Aulendorf, 3. Auflage, Band 1, S. 309) eine gewisse lokalanästhesierende Wirkung haben. Dies hat sich auch bei Versuchen zur Juckreizminderung nach Insektenstichen bestätigt (siehe Tabellen).

Dennoch waren bisherige juckreizlindernde kosmetische und/oder pharmazeutische Zusammensetzungen nicht zufriedenstellend, weil ihre Wirkungen rasch nachlassen.

Die Aufgabe der vorliegenden Erfindung bestand darin, juckreizlindernde kosmetische und/oder pharmazeutische Zusammensetzungen zur Anwendung auf der menschlichen oder tierischen Haut zur Verfügung zu stellen, die ein Anschwellen der Haut mildern, angeschwollene Haut abschwellen lassen, Rötungen verhindern und schließlich den Juckreiz über einen längeren Zeitraum lindern.

Darüber hinaus sollte sichergestellt sein, daß die erfindungsgemäßen juckreizlindernden, kosmetischen und/oder pharmazeutischen Zusammensetzungen
1. die biologischen Vorgänge der Haut nicht beeinträchtigen,
2. die Wirkstoffe keinen ausgeprägten Eigengeruch besitzen,
3. die Wirkstoffe bei Überdosierung oder sonstiger nicht bestimmungsgemäßer Anwendung unschädlich sind,
4. die Wirkstoffe sich nach wiederholter Anwendung nicht in oder auf der Haut anreichern,
5. die wirksamen Prinzipien sich gut in übliche kosmetische oder dermatologische Formulierungen einarbeiten lassen, z.B. Lösungen, Gele, Balme, Cremes, Schäume oder Stifte,
6. die Wirkstoffe die Haut nicht reizen,
7. die Wirkstoffe nicht mit Kleidungsstoffen in Wechselwirkungen treten.

Es wurde nun gefunden und darin liegt die Lösung all der obengenannten Aufgaben, daß die Verwendung eines Wirkkomplexes, bestehend aus einem oder mehreren leichten Lokalanästhetika, einer oder mehrerer Adstringentien, z.B. dem Gerbstoff Tannin, und/oder gegebenenfalls einer antiinflammatorisch wirkenden Substanz weitaus länger anhielt, als dies von der Summe der Wirksamkeit der Einzelkomponenten her zu erwarten war. Während die durchschnittliche juckreizmindernde Wirkung von Laureth-9 etwa 30 Minuten anhielt, Tannin alleine die Haut zwar abschwellen ließ und die Rötung milderte, jedoch gegen den Juckreiz nur unmittelbar (nach der Applikation weniger als 10 Minuten) wirkte, und Glycyrrhizinat und Bisabolol auch nur etwa 5 Minuten gegen den Juckreiz wirkten, hielt die Wirkung der Kombination der Einzelkomponenten bis zu 3 Stunden (Durchschnitt je 5 Probanden: 130 bis 140 Minuten) an.

Die erfindungsgemäßen Zusammensetzungen eignen sich daher hervorragend zur Linderung von Juckreiz z.B. nach Insektenstichen, Kontakt mit Brennesseln, Quallen und bei allergischen Hautreaktionen. Sie wirken der Hautrötung entgegen und zusätzlich abschwellend. Darüber hinaus wurde gefunden, daß die erfindungsgemäßen kosmetischen und/oder pharmazeutischen Dermatologika eine starke Wirkung gegen die Erythembildung nach übermäßiger UV-Bestrahlung zeigen.

Bevorzugte erfindungsgemäße Zusammensetzungen bestehen aus
1. einem oder mehreren Lokalanästhetika der Reihe Laureth-9, Polyoxyethylen-(20)-Sorbitanmonolaurat, Triethylcitrat, Acetyltriethylcitrat, Tributylcitrat oder Acetyltributylcitrat,
2. einem oder mehreren Adstringentien aus der Reihe Tannin, Walnußblätterextrakt, Aluminiumlactat oder Natriumtartrat und/oder gegebenenfalls
3. einer oder mehrerer antiinflammatorisch wirkenden Substanzen aus der Reihe Dragosantol®, Bisabolol, Panthenol, Panthothenylalkohol, Glycyrrhetin-Derivate, Glycyrrhizin-Derivate oder Lakritzextrakt.

Besonders bevorzugte erfindungsgemäße Zusammensetzungen bestehen aus
1. einem oder mehreren Lokalanästhetika der Reihe Laureth-9, Polyoxyethylen-(20)-Sorbitanmonolaurat, Triethylcitrat, Acetyltriethylcitrat, Tributylcitrat oder Acetyltributylcitrat,
2. einem oder mehreren Adstringentien aus der Reihe Tannin, Walnußblätterextrakt, Aluminiumlactat oder Natriumtartrat und/oder gegebenenfalls
3. einer oder mehrerer antiinflammatorisch wirkenden Substanzen aus der Reihe Dragosantol®, Bisabolol, Panthenol, Dexpanthenol, Panthothenylalkohol, Dikaliumglycyrrhizinat, Glycyrrhizinsäure oder deren Salze, Glycyrrhetinsäure oder deren Salze, Stearylglycyrrhetinat oder Lakritzextrakt.

Insbesondere bevorzugt ist der Wirkkomplex bestehend aus Laureth-9, Tannin, Dikaliumglycyrrhizinat und/oder Bisabolol.

Alle Komponenten sind aus der Literatur bekannt. Im einzelnen seien einige Inhaltsstoffe kurz beschrieben:

Laureth-9 ist ein Polyethylenglykol(9)monododecylether (Polidocanol), ein Addukt von 9 Mol Ethylenoxid an den Dodecylalkohol; es entspricht der Formel

C₁₃H₂₅-(O-CH₂-CH₂)ₙ-OH

(n hat einen Durchschnittswert von 9), das durchschnittliche Molgewicht beträgt etwa 600.

Dragosantol® ist eine Handelsbezeichnung für eine synthetisch hergestellte, farblose bis schwach gelblich gefärbte, schwach riechende Flüssigkeit,
(-)-α-Bisabolol [(-)-6-Methyl-2-(4-methyl-3-cyclohexenyl)-5-hepten-2-ol] 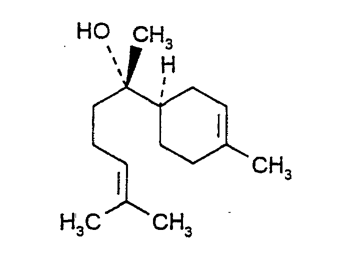 Glycyrrhetinsäure (3β-Hydroxy-11-oxoolean-12-en-30-säure, Freinamen: Enoxolon, Bioson). 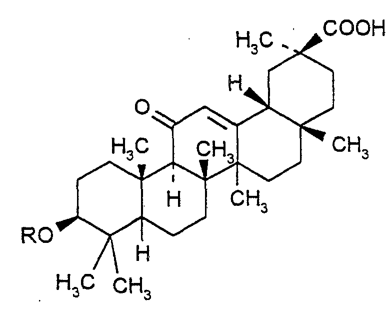 R = H: Glycyrrhetinsäure 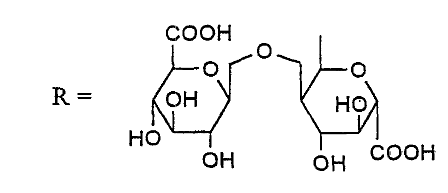 Glycyrrhizin

Die erfindungsgemäßen, juckreizlindernden, kosmetischen und/oder pharmakologischen Zusammensetzungen enthalten die für eine topische Anwendung üblichen Zusatzstoffe und Lösungen vorzugsweise als flüssige, halbfeste oder feste Zubereitungen.

Für die flüssige Zubereitung kommen Tropfen, Tinkturen oder Sprays in Frage, die jeweils die zuvor beschriebenen Wirkstoffmischungen in Form einer Lösung, Suspension, Emulsion oder Dispersion enthalten.

Als halbfeste Zubereitungen kommen beispielsweise Gele, Salben, Cremes oder Schäume in Frage, während unter feste Zubereitungen beispielsweise Pulver, Puder, Granulate, Pellets oder Mikrokapseln fallen. Werden die zuvor beschriebenen Wirkstoffmischungen in der Form einer flüssigen Darreichungsform als pharmazeutisches und/oder kosmetisches Mittel angeboten, so empfiehlt es sich, hierfür möglichst solche Verdünnungsmittel zu verwenden, die die Haut bei einer topischen Anwendung nicht reizen. Dies trifft beispielsweise insbesondere auf Wasser, einwertige Alkohole, vorzugsweise Ethanol, Isopropanol oder n-Propanol, mehrwertige Alkohole, insbesondere Glycerin und/oder Propandiol, Polyglykole, insbesondere Polyethylenglykole und/oder Miglyol, Glycerinformal, Dimethylisosorbit, natürliche und synthetische Öle und/oder Ester, zu.

Für die Herstellung von halbfesten Darreichungsformen, wie beispielsweise Gele, Salben, Cremes und Schäume, eignen sich neben den zuvor genannten Verdünnungsmitteln zusätzlich noch Grundmassen, wie beispielsweise Bentonit, Veegum, Guarmehl und/oder Cellulosederivate, insbesondere Methylcellulose und/oder Carboxymethylcellulose. Ebenso kommen als Grundmasse anstelle der zuvor genannten Grundmassen oder zusätzlich zu den zuvor genannten Grundmassen Polymere aus Vinylalkoholen, Vinylpyrrolidonen, Alginaten, Pektinen, Polyacrylaten, festen und/oder flüssigen Polyethylenglykolen, Paraffinen, Fettalkoholen, Vaselin, Wachsen, Fettsäuren und/oder Fettsäsureestern in Frage.

Für die Herstellung von festen, ebenfalls zur topischen Anwendung geeigneten Zubereitungen, wie beispielsweise die zuvor genannten Pulver, Puder, Granulate, Pellets oder Mikrokapseln, besteht die Möglichkeit, hier als Bindemittel beispielsweise kolloidale Kieselsäure, Talkum, Milchzucker, Stärkepulver, Zucker, Cellulosederivate, Gelatine, Metalloxide und/oder Metallsalze zu verwenden.

Darüber hinaus können die erfindungsgemäßen Zusammensetzungen wahlweise noch weitere Bestandteile, wie beispielsweise Konservierungsmittel, Stabilisatoren, Tenside, Emulgatoren, Penetrationsförderer, Spreitungsmittel und/oder Treibmittel, enthalten.

Die erfindungsgemäßen Mittel lassen sich gegen die Begleiterscheinungen allergischer Hautreaktionen und der Neurodermitis, sowie zur Linderung der Beschwerden nach Kontakt mit z.B. Brennesseln oder Quallen einsetzen.

Insbesondere jedoch bei Insektenstichen, so z.B. Bienenstichen, Wespenstichen, Hornissenstichen, Mückenstichen, Milbenstichen, Zeckenbissen, Sandflohbissen, Stechfliegenbissen und Bremsenbissen, konnte festgestellt werden, daß bereits nach kürzester Zeit nach Auftragen der erfindungsgemäßen Zusammensetzungen ein deutlicher Rückgang der durch die zuvor genannten Stiche bzw. Bisse hervorgerufenen Rötungen, Schwellungen und/oder Quaddelbildungen auftritt, während gleichzeitig der durch den Insektenstich bzw. Insektenbiß hervorgerufene Schmerzreiz bzw. Juckreiz deutlich reduziert wird.

Selbst bei sehr empfindlichen Patienten konnte bei einer topischen Anwendung der erfindungsgemäßen Zusammensetzungen keine unerwünschte Reizung oder Rötung der Haut festgestellt werden.

Die erfindungsgemäßen kosmetischen und/oder pharmazeutischen Zusammensetzungen können in verschiedenen Formen vorliegen, wie sie z.B. üblicherweise für diesen Typ von Zubereitungen eingesetzt werden. So können sie z.B. eine wäßrige, alkoholische oder wäßrig-alkoholische Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol darstellen.

Die erfindungsgemäßen Zusammensetzungen können kosmetische Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die erfindungsgemäßen kosmetischen und/oder pharmazeutischen Zusammensetzungen eine Lösung oder Lotion darstellen, können als Lösungsmittel verwendet werden:
- Wasser oder wäßrige Lösungen;
- Öle, wie Triglyceride der Caprin- oder der Caprylsäure, vorzugsweise aber Pflanzenöle wie z.B. Rizinusöl, Rapsöl, etc;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl-, -monoethyl- oder -monobutylether, Diethylenglykolmonomethyloder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die erfindungsgemäßen kosmetischen und/oder pharmazeutischen Zusammensetzungen können auch als Gele oder Hydrodispersionen vorliegen, die neben den erfindungsgemäßen Wirkstoffkombinationen noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Erfindungsgemäße Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatz zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen, metastabile Systeme dar und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Feste Stifte gemäß der Erfindung können z.B. natürliche oder synthetische Wachse, Fettalkohole oder Fettsäureester enthalten.

Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es nicht-toxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die erfindungsgemäßen kosmetischen und/oder pharmazeutischen Zusammensetzungen enthalten neben den für Hautpflegemittel üblichen Grund- und Hilfsstoffen bevorzugt
0,1 bis 30 % Lokalanästhetika, z.B. Laureth-9
0,1 bis 30 % Adstringentien z.B. Tannin sowie gegebenenfalls
0,05 bis 10 % antiinflammatorisch wirkende Substanzen wie z.B. Bisabolol und/oder Dikalium-Glycyrrhizinat.

Besonders bevorzugt enthalten die erfindungsgemäßen kosmetischen und/oder pharmazeutischen Zusammensetzungen neben den für Hautpflegemittel üblichen Grund- und Hilfsstoffen
0,1 bis 20 % Lokalanästhetika,
0,1 bis 20 % Adstringentien,
0,05 bis 10 % eines oder mehrere antiinflammatorisch wirkende Substanzen.

Des weiteren enthalten die erfindungsgemäßen Zusammensetzungen Grund- und Hilfsstoffe.

Zu den Grund- und Hilfsstoffen zählen in der Kosmetik bereits obengenannte übliche Lösungsmittel wie Wasser bis zu 70 %, Monoalkohole, niedrige Polyalkohole mit 1 bis 6 Kohlenstoffatomen oder Mischungen davon, weiterhin Fettkörper, wie mineralische, tierische, oder pflanzliche Öle wie Paraffinöl oder Wachse wie Mikrowachs, Fettsäuren, Fettalkohole, Fettsäureester wie Cetylstearylisononanoat und Isopropylpalmitat, Fettalkoholether, oxethylierte Fettalkohole, Lanolin und Derivate sowie Silikonöle in Mengen von 0,5 bis 50 %, vorzugsweise 0,5 bis 30 %, besonders bevorzugt in Mengen von 5 bis 30 %.

Die erfindungsgemäßen Zusammensetzungen enthalten gegebenenfalls Emulgatoren in Mengen von 0,1 bis 20 %, bevorzugt in Mengen von 0,2 bis 10 %, wobei es sich um Emulgatoren handelt, wie sie in der Kosmetik üblicherweise verwendet werden, insbesondere um nichtionische, anionische, kationische oder amphotere Verbindungen, z.B. Sterole, Polyol-Fettsäureester und -Fettalkoholether, Alkaliund Triethanolaminsalze von Fettsäuren, Natriumcetylstearylsulfat, Tetraacylammoniumhalogenide, Phospholipide. Beispiele hierfür sind Glycerinsorbitanfettsäureester, Polyoxyethylenfettsäureester, Alkyltetraglykolether-o-phosphorsäureester.

Ferner können 0,02 bis 5 %, bevorzugt 0,1 bis 2 % Verdickungsmittel und Vergelungsmittel in den erfindungsgemäßen Zusammensetzungen eingesetzt werden. Dazu zählen Polyacrylsäurederivate, Cellulosederivate, Bentonite, Xanthanderivate, Alginate, Guarmehl und Johannisbrotmehl. Beispiele sind Polyacrylsäureamid und Zinkstearat.

Das erfindungsgemäße Präparat kann, wie bereits oben erwähnt, weitere in kosmetischen Mitteln übliche Stoffe enthalten. Dazu zählen Feuchthaltemittel (0,5 bis 15 %), Farbstoffe, Puffersubstanzen, Konservierungsmittel und Parfümöle in Mengen von 0,01 bis 5,0 %.

Als Feuchthaltemittel seien beispielhaft aufgeführt: Niedrige Polyalkohole wie Glycerin, Propylenglykol, Butylenglykol, Sorbitol, desweiteren die 2-Pyrrolidon-5-carbonsäure und ihr Natriumsalz, Milchsäure und ihre Salze, Harnstoff, Proteine und Proteinderivate wie Collagen, desweiteren Hyaluronsäure u.a.

Als den erfindungsgemäßen kosmetischen und/oder pharmazeutischen Präparaten zuzusetzende Farbstoffe seien beispielhaft aufgeführt:
Farbe C.I. 16255, Farbe C.I. 61570, Farbe C.I. 42051, Farbe C.I. 15985, Farbe C.I. 77492.

Als Konservierungsmittel kommen vorzugsweise in Frage:
2,4-Hexadiensäure (Sorbinsäure und ihre Salze),
4-Hydroxybenzoesäure, ihre Salze und Ester,
3-Acetyl-6-methyl-2,4(3H)-pyrandion (Dehydracetsäure) und seine Salze,
1,1-Methylen-bis-(3-(1-hydroxymethyl-2,4-dioximidazolidin-5-yl)-harnstoff), Imidazolidinylhamstoff,
2-Phenoxy-ethanol,
Benzylalkohol.

Die erfindungsgemäßen Zusammensetzungen liegen vorzugsweise als Lösung oder als Emulsion (Creme oder Milch) vor, wobei es sich um Öl-in-Wasser- oder Wasser-in-Öl-Emulsionen handeln kann.

Die erfindungsgemäßen Zusammensetzungen werden nach üblichen Verfahren hergestellt. Dabei werden die eigentlichen Wirksubstanzen vorgelegt und mit den üblichen Grund- und Hilfsstoffen, gegebenenfalls unter Vordispergieren, Rühren und/oder Homogenisieren, gegebenenfalls in einer evakuierten Apparatur, vermischt.

Alle Prozentangaben im vorliegenden Text beziehen sich auf Gewichtsprozente, wenn nichts anderes angegeben ist.

Die Erfindung wird im folgenden anhand der Beispiele näher erläutert, ohne daß dies einschränkenden Charakter haben soll.

### Rezepturbeispiele

### Beispiel 1

Juckreizlinderndes Präparat enthaltend
0,5 bis 20 % Laureth-9
0,1 bis 15 % Tannin und evtl.
0,05 bis 3 % Dikalium Glycyrrhizinat und/oder
0,05 bis 3 % Bisabolol.

### Beispiel 2

Rezeptur für eine juckreizlindernde Lösung

| | |
|---|---|
| Laureth-9 | 3,50 % |
| Tanninsäure | 5,00 % |
| Bisabolol | 0,40 % |
| Ethanol | 30,00 % |
| 1,3-Butylenglykol | 5,00 % |
| Parfüm | q.s. |
| Wasser | ad 100,00 % |

### Beispiel 3

Rezeptur für ein juckreizlinderndes Gel

| | |
|---|---|
| Laureth-9 | 6,00 % |
| Tanninsäure | 1,50 % |
| Dikalium Glycyrrhizinat | 1,00 % |
| Carbopol 2984® | 0,70 % |
| Natronlauge 45 %ig | 0,05 % |
| Ethanol | 30,00 % |
| Glycerin | 10,00 % |
| Parfüm/Konservierungsmittel | q.s. |
| Wasser | ad 100,00 % |

### Wirksamkeitstest

Bei freiwilligen Personen wurden unter kontrollierten Bedingungen an beiden dorsalen Unterarmen Mückenstiche (Aedes aegypti) provoziert. Im Blindtest wurde jeweils ein Arm mit Placebo und ein Arm mit Wirkstofflösung behandelt. Die Versuchspersonen beurteilen die Versuchsprodukte hinsichtlich der Dauer der Juckreizlinderung.

### Rezepturen

### A: Placebo (Basisrezeptur)

| | |
|---|---|
| Wasser | 57,00 % |
| Glycerin | 3,00 % |
| Ethanol | 40,00 % |

### B: Basisrezeptur + Laureth-9

| | |
|---|---|
| Wasser | 57,00 % |
| Glycerin | 3,00 % |
| Ethanol | 40,00 % |
| Laureth-9 | 5,00 % |

### C: Basisrezeptur + Tannin

| | |
|---|---|
| Wasser | 55,00 % |
| Glycerin | 3,00 % |
| Ethanol | 40,00 % |
| Tanninsäure | 2,0 % |

### D: Basisrezeptur + Dikaliumglycyrrhizinat

| | |
|---|---|
| Wasser | 56,90 % |
| Glycerin | 3,00 % |
| Ethanol | 40,00 % |
| Dikaliumglycyrrhizinat | 0,10 % |

### E: Basisrezeptur + Bisabolol

| | |
|---|---|
| Wasser | 56,90 % |
| Glycerin | 3,00 % |
| Ethanol | 40,00 % |
| Bisabolol | 0,10 % |

### F: Kombination 1 dieser Wirkstoffe

| | |
|---|---|
| Wasser | 49,80 % |
| Glycerin | 3,00 % |
| Ethanol | 40,00 % |
| Laureth-9 | 5,00 % |
| Tanninsäure | 2,00 % |
| Dikaliumglycyrrhizinat | 0,10 % |

### G: Kombination 2 dieser Wirkstoffe

| | |
|---|---|
| Wasser | 49,80 % |
| Glycerin | 3,00 % |
| Ethanol | 40,00 % |
| Laureth-9 | 5,00 % |
| Tanninsäure | 2,00 % |
| Bisabolol | 0,10 % |

### Ergebnisse Dauer der Juckreizlinderung

| Proband | A: Placebo in min | B: Laureth 9 in min |
|---|---|---|
| 1 | 0 | 25 |
| 2 | 10 | 30 |
| 3 | 0 | 60 |
| 4 | 5 | 15 |
| 5 | 10 | 20 |
| Durchschnitt | 5 | 30 |

| Proband | A: Placebo in min | C: Tannin in min |
|---|---|---|
| 1 | 5 | 0 |
| 2 | 0 | 15 |
| 3 | 0 | 5 |
| 4 | 10 | 20 |
| 5 | 0 | 5 |
| Durchschnitt | 3 | 9 |

| Proband | A: Placebo in min | D: Dikalium Glycyrrhizinat in min |
|---|---|---|
| 1 | 0 | 10 |
| 2 | 10 | 5 |
| 3 | 0 | 0 |
| 4 | 10 | 15 |
| 5 | 5 | 10 |
| Durchschnitt | 5 | 8 |

| Proband | A: Placebo in min | E: Bisabolol in min |
|---|---|---|
| 1 | 10 | 10 |
| 2 | 0 | 5 |
| 3 | 15 | 5 |
| 4 | 15 | 5 |
| 5 | 0 | 5 |
| Durchschnitt | 8 | 6 |

| Proband | A: Placebo in min | F: Kombination 1 in min |
|---|---|---|
| 1 | 0 | 90 |
| 2 | 10 | 120 |
| 3 | 15 | 190 |
| 4 | 0 | 160 |
| 5 | 0 | 90 |
| Durchschnitt | 5 | 130 |

| Proband | A: Placebo in min | G: Kombination 2 in min |
|---|---|---|
| 1 | 0 | 115 |
| 2 | 10 | 90 |
| 3 | 15 | 210 |
| 4 | 0 | 160 |
| 5 | 0 | 125 |
| Durchschnitt | 5 | 140 |

## Patentansprüche

1. Juckreizlindernde, kosmetische und/oder pharmazeutische Zusammensetzungen, **dadurch gekennzeichnet, daß** sie aus einem oder mehreren Lokalanästhetika, einem oder mehreren Adstringentien und/oder gegebenenfalls einer antiinflammatorisch wirkenden Substanz bestehen.

2. Juckreizlindernde, kosmetische und/oder pharmazeutische Zusammensetzungen, **dadurch gekennzeichnet, daß** sie aus
einem oder mehreren Lokalanästhetika der Reihe Laureth-9, Polyoxyethylen-(20)-Sorbitanmonolaurat, Triethylcitrat, Acetyltriethylcitrat, Tributylcitrat oder Acetyltributylcitrat,
einem oder mehreren Adstringentien aus der Reihe Tannin, Walnußblätterextrakt, Aluminiumlactat oder Natriumtartrat und/oder gegebenenfalls
einer oder mehrerer antiinflammatorisch wirkenden Substanzen aus der Reihe Dragosantol®, Bisabolol, Panthenol, Panthethenylalkohol, Glycyrrhetin-Derivate, Glycyrrhizin-Derivate oder Lakritzextrakt bestehen.

3. Juckreizlindernde, kosmetische und/oder pharmazeutische Zusammensetzungen, **dadurch gekennzeichnet, daß** sie aus
einem oder mehreren Lokalanästhetika der Reihe Laureth-9, Polyoxyethylen-(20)-Sorbitanmonolaurat, Triethylcitrat, Acetyltriethylcitrat, Tributylcitrat oder Acetyltributylcitrat,
einem oder mehreren Adstringentien aus der Reihe Tannin, Walnußblätterextrakt, Aluminiumlactat oder Natriumtartrat und/oder gegebenenfalls
einer oder mehrerer antiinflammatorisch wirkenden Substanzen aus der Reihe Dragosantol®, Bisabolol, Panthenol, Dexpanthenol, Panthothenylalkohol, Dikaliumglycyrrhizinat, Glycyrrhizinsäure oder deren Salze, Glycyrrhetinsäure oder deren Salze, Stearylglycyrrhetinat oder Lakritzextrakt bestehen.

4. Juckreizlindernde, kosmetische und/oder pharmazeutische Zusammensetzungen, **dadurch gekennzeichnet, daß** sie aus Laureth-9, Tannin, Dikaliumglycyrrhizinat und/oder Bisabolol bestehen.

5. Juckreizlindernde, kosmetische und/oder pharmazeutische Zusammensetzungen gemäß den Ansprüchen 1 bis 4, **dadurch gekennzeichnet, daß** sie als flüssige, halbfeste oder feste Zubereitungen vorliegen.

6. Verwendung der juckreizlindernden, kosmetischen und/oder pharmazeutischen Zusammensetzungen der Ansprüche 1 bis 5 zur Herstellung eines Arzneimittels zur therapeutischen Behandlung von Insektenstichen und durch Licht und/oder Wärme hervorgerufene Hautschäden.

7. Juckreizlindernde, kosmetische und/oder pharmazeutische Zusammensetzungen gemäß den Ansprüchen 1 bis 5, **dadurch gekennzeichnet, daß** sie 0,1 bis 30 Gew.-% Lokalanästhetika, 0,1 bis 30 Gew.-% Adstringentien sowie gegebenenfalls 0,05 bis 10 Gew.-% antiinflammatorisch wirkende Substanzen enthalten.

8. Juckreizlindernde, kosmetische und/oder pharmazeutische Zusammensetzungen gemäß den Ansprüchen 1 bis 5, **dadurch gekennzeichnet, daß** sie neben den eigentlichen Wirkstoffen noch zusätzlich Grund- und Hilfsstoffe, Emulgatoren, Verdickungsmittel, Vergelungsmittel, Feuchthaltemittel, Farbstoffe, Puffersubstanzen, Konservierungsmittel, Parfümöle, Stabilisatoren, Tenside, Spreitungsmittel und/oder Treibmittel enthalten.

9. Verfahren zur Herstellung der juckreizlindernden, kosmetischen und/oder pharmazeutischen Zusammensetzungen gemäß den Ansprüchen 1 bis 5, **dadurch gekennzeichnet, daß** man die eigentlichen Wirksubstanzen vorlegt und mit den üblichen Grund- und Hilfsstoffen, gegebenenfalls unter Vordispergieren, Rühren und/oder Homogenisieren, gegebenenfalls in einer evakuierten Apparatur, vermischt.

## Claims

1. Antipruritic cosmetic and/or pharmaceutical compositions, **characterized in that** they comprise one or more local anaesthetics, one or more astringents and/or, if appropriate, a substance having an antiinflammatory action.

2. Antipruritic cosmetic and/or pharmaceutical compositions, **characterized in that** they comprise
one or more local anesthetics of the series consisting of laureth-9, polyoxyethylene (20)-sorbitan monolaurate, triethyl citrate, acetyl-triethyl citrate, tributyl citrate or acetyl-tributyl citrate,
one or more astringents from the series consisting of tannin, walnut leaf extract, aluminium lactate or sodium tartrate and/or, if appropriate,
one or more substances having an antiinflammatory action from the series consisting of Dragosantol®, bisabolol, panthenol, pantothenyl alcohol, glycyrrhetin derivatives, glycyrrhizin derivatives or liquorice extract.

3. Antipruritic cosmetic and/or pharmaceutical compositions, **characterized in that** they comprise
one or more local anesthetics of the series consisting of laureth-9, polyoxyethylene (20)-sorbitan monolaurate, triethyl citrate, acetyl-triethyl citrate, tributyl citrate or acetyl-tributyl citrate,
one or more astringents from the series consisting of tannin, walnut leaf extract, aluminum lactate or sodium tartrate and/or, if appropriate,
one or more substances having an antiinflammatory action from the series consisting of Dragosantol®, bisabolol, panthenol, dexpanthenol, pantothenyl alcohol, dipotassium glycyrrhizinate, glycyrrhizinic acid or salts thereof, glycyrrhetic acid or salts thereof, stearyl glycyrrhetinate or liquorice extract.

4. Antipruritic, cosmetic and/or pharmaceutical compositions, **characterized in that** they comprise laureth-9, tannin, dipotassium glycyrrhizinate and/or bisabolol.

5. Antipruritic cosmetic and/or pharmaceutical compositions according to Claims 1 to 4, **characterized in that** they are in the form of liquid, semisolid or solid formulations.

6. Use of the antipruritic cosmetic and/or pharmaceutical compositions of Claims 1 to 5 for the preparation of a medicament for therapeutic treatment of insect bites and skin damage caused by light and/or heat.

7. Antipruritic cosmetic and/or pharmaceutical compositions according to Claims 1 to 5, **characterized in that** they comprise 0.1 to 30 % by weight of local anesthetics, 0.1 to 30 % by weight of astringents and, if appropriate, 0.05 to 10 % by weight of substances having an antiinflammatory action.

8. Antipruritic cosmetic and/or pharmaceutical compositions according to Claims 1 to 5, **characterized in that**, in addition to the actual active compounds, they also additionally comprise base substances and auxiliaries, emulsifiers, thickeners, gelling agents, humectants, dyestuffs, buffer substances, preservatives, perfume oils, stabilizers, surfactants, spreading agents and/or propellants.

9. Process for the preparation of the antipruritic cosmetic and/or pharmaceutical compositions according to Claims 1 to 5, **characterized in that** the actual active substances are initially introduced into the preparation vessel and are mixed with the customary base substances and auxiliaries, if appropriate with predispersion, stirring and/or homogenization, if appropriate in an evacuated apparatus.

## Revendications

1. Compositions cosmétiques et/ou pharmaceutiques antiprurigineuses, **caractérisées en ce qu'**elles consistent en un ou plusieurs anesthésiques locaux, un ou plusieurs astringents, et/ou le cas échéant, une substance anti-inflammatoire.

2. Compositions cosmétiques et/ou pharmaceutiques antiprurigineuses, **caractérisées en ce qu'**elles consistent en
un ou plusieurs anesthésiques locaux de la série du laureth-9, du monolaurate de sorbitan-polyoxyéthylène (20), du citrate de triéthyle, du citrate d'acétyltriéthyle, du citrate de tributyle ou du citrate d'acétyltributyle ;
un ou plusieurs astringents de la série de l'acide tannique, de l'extrait de feuille de noix, du lactate d'aluminium ou du tartrate de sodium, et/ou le cas échéant,
une ou plusieurs substances anti-inflammatoires de la série du Dragosantol®, du bisabolol, du panthénol, de l'alcool panthothénylique, des dérivés de l'acide glycyrrhétique, des dérivés de l'acide glycyrrhizique ou de l'extrait de réglisse.

3. Compositions cosmétiques et/ou pharmaceutiques antiprurigineuses, **caractérisées en ce qu'**elles consistent en
un ou plusieurs anesthésiques locaux de la série du laureth-9, du monolaurate de sorbitan-polyoxyéthylène (20), du citrate de triéthyle, du citrate d'acétyltriéthyle, du citrate de tributyle ou du citrate d'acétyltributyle ;
un ou plusieurs astringents de la série de l'acide tannique, de l'extrait de feuille de noix, du lactate d'aluminium ou du tartrate de sodium, et/ou le cas échéant,
une ou plusieurs substances anti-inflammatoires de la série du Dragosantol®, du bisabolol, du panthénol, du dexpanthénol, de l'alcool panthothénylique, du glycyrrhizinate de dipotassium, de l'acide glycyrrhizique ou de ses sels, de l'acide glycyrrhétinique ou de ses sels, du glycyrrhéthinate de stéaryle ou de l'extrait de réglisse.

4. Compositions cosmétiques et/ou pharmaceutiques antiprurigineuses, **caractérisées en ce qu'**elles consistent en le laureth-9, l'acide tannique, le glycyrrhizinate de dipotassium et/ou le bisabolol.

5. Compositions cosmétiques et/ou pharmaceutiques antiprurigineuses, suivant les revendications 1 à 4, **caractérisées en ce qu'**elles se présentent sous la forme de préparations liquides, semi-solides ou solides.

6. Utilisation des compositions cosmétiques et/ou pharmaceutiques antiprurigineuses, suivant les revendications 1 à 5, pour la préparation d'un agent pharmaceutique pour le traitement thérapeutique des piqûres d'insecte et des lésions de la peau produites par la lumière et/ou la chaleur.

7. Compositions cosmétiques et/ou pharmaceutiques antiprurigineuses, suivant les revendications 1 à 5, **caractérisées en ce qu'**elles contiennent 0,1 à 30% en poids d'anesthésique local, 0,1 à 30% en poids d'astringent, ainsi que le cas échéant 0,05 à 10% en poids d'une substance anti-inflammatoire.

8. Compositions cosmétiques et/ou pharmaceutiques antiprurigineuses, suivant les revendications 1 à 5, **caractérisées en ce qu'**elles contiennent en plus des agents actifs individuels, des agents de base et auxiliaires, des émulsionnants, des agents épaississants, des agents d'allongement, des substances humidifiantes, des colorants, des substances tampon, des agents de conservation, de huiles parfumées, des stabilisants, des tensioactifs, des agents d'étalement et/ou des agents propulseurs.

9. Procédé de préparation des comppsitions cosmétiques et/ou pharmaceutiques antiprurigineuses, suivant les revendications 1 à 5, **caractérisé en ce que** les substances actives réelles sont disposées et mélangées avec les agents de base et auxiliaires usuels, le cas échéant par dispersion, agitation et/ou homogénéisation, éventuellement. dans un appareil mis sous vide.
